# EUROPEAN PATENT APPLICATION

(11) **EP 0 959 079 A1**
(43) Date of publication of application: **24.11.1999**
(21) Application number: 97947684.3
(22) Date of filing: 17.12.1997
(51) Int. Cl.: C07K 7/23, A61K 38/09

(54) **VACCINE FOR THE REVERSIBLE IMMUNOCASTRATION OF MAMMALS**

(30) Priority: 17.12.1996 CU 12096
(71) Applicant: CENTRO DE INGENIERIA GENETICA Y BIOTECNOLOGIA (CIGB), Ciudad de la Habana 10600 (CU)
(72) Inventor: BRINGAS PEREZ, Ricardo-Apartamento 32, Ciudad de la Habana 10600 (CU); BASULTO BAKER,Roberto;-Calle Sociedad Patriútica 6, Camaguey, CP 70300 (CU); REYES ACOSTA, Osvaldo-Calle Carmen 54, Ciudad de la Habana 10700 (CU); DE LA FUENTE GARCIA, José-Apartamento 38, Ciudad de la Habana 10600 (CU)
(74) Representative: Ottevangers, Sietse Ulbe
(86) International application number: CU9700008
(87) International publication number: WO9827111

(57) **Abstract**

The invention relates to the field of genetic engineering and biotechnology and particularly to the use of a peptide derived from GnRH for the immunocastration of mammals. Vaccinations tests have been carried out in mammals with the gonadotropine release hormone (GnRH), in order to ellicit and immune response which neutralizes to activity of said hormone. The present invention relates to the use of a GnRH variant of mammals wherein a glycin residue has been substituted by a prolin residue in position 6: Glu-His-Trp-Ser-Tyr-Pro-Leu-Arg-Pro-Gly. This substitution or change has induced an immune response which is superior than that of natural GnRH, when both are coupled to a same carrier protein, in experiments carried out in pigs. This same result can be expected in any other mammal since this hormone is present in all species.

## Description

### Technical branch

The present invention is related with the field of Genetic Engineering and Biotechnology and in particular with the use of a peptide derived from GnRH for the immunocastration of mammals.

### Previous technique

In domestic animals, like dogs, cats, pigs and cows, it is frequent the convenience of preventing the reproduction. The methods more frequently used are the surgical castration and the administration of steroids. Both methods have inconveniences, the former requires of specialized personnel and is irreversible and the later causes side effects. For these reasons the immunocastration of animals with the use of GnRH-like peptides conjugated with proteins has been assayed, as an alternative procedure, inducing an antibody response that neutralizes the function of the GnRH.

### Detailed description of the invention

The primary structure of GnRH of several species is known. All the sequences of mammals reported so far are identical:
Glu-His-Trp-Ser-Tyr-Gly-Leu-Arg-Pro-Gly

For this reason in the literature it is referred as mammalian GnRH. In most of the known sequences, including sequences from birds, fishes and reptiles, in the position 6 is found a glycine residue. Glycine is the minor of the aminoacids and provokes flexibility in the peptide chain. The substitution of glycine by a proline, a residue that confers rigidity, could increase the immune response. Additionally, this change would provoke that this peptide would be seen as non-self for the immune system, stimulating the antibody response.

The antibody response against the GnRH is achieved conjugating the GnRH-like peptide to a carrier protein. The conjugation can be carried out by chemical or Genetic Engineering methods. In this work were chosen two proteins widely used for these purpose, BSA protein and an epitope of the tetanus toxoide. BSA is conjugated by a well-known chemical method, the epitope of the tetanus toxoide and the GnRH-like peptide are synthesized in tandem using two glycine residues as linkers. In this case the peptide is chemically synthesized as the size of the peptide is only 27 aminoacids. In case of large peptides the most feasible way for the production would be Genetic Engineering methods, inserting the GnRH-like peptide as part of the carrier protein. This is valid for any protein, for this reason it is necessary to protect the present invention from any protein obtained by chemical synthesis or genetic manipulation which contains the claimed sequences.

### Novelty and advantages of the proposed solution

Previously there have been introduced changes at position six of GnRH, replacing the glycine by D-aminoacids and other aminoacid analogs (Rivier *et al*., U.S Patent 4,215,038; Voucher, Jr. *et al*., US Patent 4,410,514). The novelty of this invention consists in the replacement of glycine in position six of GnRH by the L-aminoacid proline, that is an aminoacid that naturally occurs in proteins. This variant has demonstrated to be more effective than the natural GnRH when both are conjugated to the same protein. Using for replacement an aminoacid that naturally occurs in proteins, it gives the possibility of introducing this variant in genetic constructions, something impossible in GnRH analogs that include D-aminoacids or similar compounds.

### EXAMPLES OF REALIZATION

### EXAMPLE 1

### Peptide Synthesis.

All peptides were synthesized by solid phase method and Boc/Bzl strategy using on 4-methyl-benzhydrilamine (MBHA) resin (0.75 mmol/g, BACHEM, Swiss). The Boc- protected amino acids were purchased from BACHEM. The side-chain protecting groups were Arg(Tos), Asp(OBzl), Cys(4-MeBzl), Glu(OBzl), Lys(2-Cl-Z), Trp(CHO), Tyr(Cl₂-Bzl), Thr(Bzl) and Ser(Bzl). For assembling the peptide sequences a unique protocol was followed:

| Step | Times x min. |
|---|---|
| Dichloromethane (DCM) wash | 1 x 1 |
| Deprotection of Boc-amino groups (TFA 37.5% in DCM) | 1 x 30 |
| DCM wash | 2 x 1 |
| 2-Propanol wash | 2 x 1 |
| DCM wash | 2 x 1 |
| Neutralization | 3 x 2 |
| DCM wash | 2 x 1 |
| Coupling reaction | 1 x 60 |
| Dimethylformamide (DMF) wash | 2 x 1 |
| DCM wash | 1 x 1 |

For the neutralization step 5% of N, N-ethyldiisopropylamine (DIEA) in DCM was used.
Couplings reactions were carried out *in situ* using N, N'-diisopropylcarbodiimide (DIC). In the cases of Asn and Gln amino acids DIC and 1-hydroxybenzotriazole (HOBt) in N,N-dimethylformamide were used for this reaction.
The peptides were cleaved from the resin using the standard "Low-High" HF procedure in equipment specially designed for this purpose. In the "Low" HF step, the side-chain protecting groups were eliminated using the mixture HF(25%):Dimethylsulfide(65%):p-Cresole(10%) (120 min., 0 °C). For the peptides containing Trp in the sequence, the mixture was changed to HF(25%): Dimethyisulfide (60%): Ethanedithiol(10%):p-Cresole(5%). After this step the deprotected peptide-resins were washed with diethyl ether, dicloromethane and 2-propanol several times and dried in vacuum.
In the "High" HF step, the deprotected peptide-resins were treated with the mixture HF(90%):Anisoie(10%) (60 min., 0 °C). The free peptides were extracted with 30 % acetic acid, diluted with water and lyophilized.
Finally, the peptides were characterized by rp-HPLC using an analytical C-18 VYDAC column and by FAB ionization mass spectrometry. The peptide purification was carried out with the same chromatographic method in a preparative VYDAC C-18 column.
The synthesized peptide were:
a) QHWSYGLRPG, identified as GnRH, sequence corresponding to the gonadotropin release hormone.
b) QHWSYGLRPGGGQYIKANSKFIGITEL, identified as GnRH-TT, where the gonadotropin release hormone was assembled in tandem with the Th epitope from tetanus toxoid (830-844 residues) (Panina-Bordingnon et al, 1989). Two Gly were used as linker.
c) QHWSYPLRPG, new GnRH peptide analog identified as GnRHm1, where in the GnRH sequence the Gly in the position 6 has been changed by Pro.
d) QHWSYPLRPGGGQYIKANSKFIGITEL, identified as GnRHm1-TT. In this case the analog peptide GNRHm1 is conjugated to the N-terminus of the same epitope as in peptide GnRH-TT.
In figure 2 are shown the cromatograms of peptides GnRH (figure 2a), GnRHm1 (figure 2b), GnRH-TT (figure 2c) and GnRHm1-TT (figure 2d)

### CONJUGATE PREPARATION

The conjugation of peptides to BSA was carried following the protocol:
- Choose equal quantities of BSA (*bovine serum albumin*, Sigma) and peptide (3 mg).
- Dissolve BSA in 0,5 ml of 0,1 M Na₂CO₃.
- Dissolve peptide in 0,5 mL of a solution 0,1 M of Na₂CO₃.
- Add to resuspended BSA 3 µl of glutaraldehido at 25 % (grade I, Sigma) with slow agitation and add the resuspended peptide drop by drop.
- Add 0,1 M Na₂CO₃ to complete 1,5 ml.
- Mix with slow agitation and protected from light during 12 hours at 25°C
- Dialize one time for 12 hours against PBS.
- Add PBS 1X to complete 3 ml.
- Distribute 1 ml (1 mg of conjugated peptide) to each syringe of 5 ml.
- Add 1 ml of complete Freund's adjuvant (CFA) (Sigma) for the first immunization and 1ml of incomplete Freund's adjuvant (IFA) for the second immunization.
- Agitate for 30 minutes at room temperature.
- Keep the immunogen at 4°C until its use.

### EXAMPLE 2

Vaccine formulation and vaccination.
For vaccine formulation 1mg of peptide resuspended in 1 ml of PBS 1x was emulsified with 1 ml of CFA. One mg of peptides GnRH-TT and GnRHm1-TT are equivalent to 0.4 mg of GnRH and GnRHm1, respectively. A stable water-in-oil emulsion was obtained by agitation with a agitator with impelent. In all cases the immunogens were prepared immediately before vaccination.

The second vaccination was administered 8 weeks after the first vaccination but was emulsified with IFC.
The Animals : Fifteen male piglets from different litters were assigned to 5 different groups of 3 animals.
Vaccination: The five groups were vaccinated the first time between the 11 and 12 week of age and received a booster vaccination 8 weeks later. In both cases the vaccination was administered intramuscular in the neck. All the animal were sacrificed 8 weeks after the second vaccination. The first group was vaccinated only with PBS 1x and CFA and served as negative control of the experiment. The second and third groups were vaccinated with GnRH-BSA and GnRH-TT, respectively, and were used as positive control, considering that GnRH is the endogenous hormone. The forth and fifth groups were vaccinated with the new peptide GnRHm1 conjugated to BSA (GnRHm1-BSA) and to the tetanus toxoide (GnRHm1-TT), respectively. The vaccination schedule comprised four months (from January to April, 1996)

### EXAMPLE 3

Evaluation during the vaccination schedule.
Starting from day zero and every forth week until the sacrifice the testicle size and animal weight were recorded. Serum samples were taken for determination of anti-GnRH titers. Additionally, at week 16 the reflex of sexual intercourse: approaching, covering, erection and ejaculation, were tested during three days. The stimulation was made in some cases with a female piglet in natural heat and in other case with a female in heat induced with seric gonadotropin.
a)

**Table 1**

| Testicles measurement : | | | | | | | | | | | |
|---|---|---|---|---|---|---|---|---|---|---|---|
| | | TESTICLE SIZE, mm | | | | | | | | | |
| GROUP | ANIMAL | week 0 | | week 4 | | week 8 | | week 12 | | week 16 | |
| | | R | L | R | L | R | L | R | L | R | L |
| GnRH-TT | 20 | 39 | 38 | 42 | 41 | 55 | 57 | 52 | 51 | 76 | 74 |
| | 30 | 41 | 42 | 47 | 47 | 53 | 53 | 55 | 54 | 52 | 52 |
| | 36 | 35 | 37 | 40 | 38 | 46 | 46 | 51 | 51 | 62 | 58 |
| GnRHm1-TT | 15 | 45 | 43 | 50 | 49 | 65 | 64 | 50 | 46 | 58 | 62 |
| | 24 | 38 | 37 | 32 | 32 | 43 | 45 | 42 | 40 | 47 | 50 |
| | 37 | 45 | 43 | 49 | 50 | 56 | 55 | nd | 50 | 42 | 42 |
| GnRH-BSA | 2 | 44 | 43 | 44 | 43 | 52 | 51 | 50 | 58 | 74 | 72 |
| | 23 | 47 | 45 | 48 | 46 | 64 | 62 | 90 | 90 | 120 | 120 |
| | 25 | 45 | 43 | 49 | 50 | 57 | 57 | 75 | 74 | 90 | 88 |
| GnRHm1-BSA | 14 | 45 | 43 | 41 | 42 | 63 | 63 | 89 | 92 | 96 | 96 |
| | 16 | 44 | 45 | 40 | 40 | 55 | 54 | 82 | 84 | 90 | 90 |
| | 39 | 43 | 42 | 44 | 45 | 55 | 55 | 77 | 75 | 87 | 87 |
| PLACEBO | 1 | 48 | 46 | 52 | 51 | 60 | 59 | 85 | 83 | 100 | 98 |
| | 11 | 43 | 42 | 48 | 47 | 50 | 50 | 72 | 72 | 85 | 92 |
| | 17 | 48 | 46 | 50 | 49 | 62 | 61 | 82 | 80 | 98 | 105 |
| nd- not determined for the impossibility to localize the testicle. R and L- right and left testicle, respectively. | | | | | | | | | | | |

b)

**Table 2**

| Pig's weight : | | | | | | |
|---|---|---|---|---|---|---|
| **GROUP** | **ANIMAL** | **Body weight, Kg** | | | | |
| | | **Week 0** | **week 4** | **week 8** | **week 12** | **week 16** |
| **GnRH-TT** | 20 | 10,6 | Nd | 29,5 | 42,0 | 54,0 |
| | 30 | 10,0 | 15,6 | 28,5 | 40,0 | 55,5 |
| | 36 | 10,8 | Nd | 30,0 | 41,5 | 55,5 |
| **GnRHm1-TT** | 15 | 12,6 | Nd | 33,5 | 43,0 | 58,0 |
| | 24 | 12,6 | Nd | 33,0 | 43,0 | 59,0 |
| | 37 | 13,2 | Nd | 37,0 | 50,0 | 61,5 |
| **GnRH-BSA** | 2 | 13,4 | Nd | 35,2 | 47,5 | 64,0 |
| | 23 | 13,0 | Nd | 33,0 | 47,5 | 61,0 |
| | 25 | 13,6 | nd | 32,7 | 40,5 | 48,0 |
| **GnRHm1-BSA** | 14 | 12,0 | nd | 30,5 | 40,0 | 56,0 |
| | 16 | 12,9 | nd | 27,5 | 42,5 | 48,0 |
| | 39 | 13,1 | 17,5 | 30,6 | 43,5 | 58,0 |
| **PLACEBO** | 1 | 16,0 | nd | 37,3 | 45,5 | 57,0 |
| | 11 | 14,5 | nd | 38,5 | 49,0 | 62,0 |
| | 17 | 17,0 | 24 | 39,5 | 55,0 | 70,0 |
| nd- not determined. | | | | | | |

c)

**Table 3**

| Growth of body weight for each group. | | | |
|---|---|---|---|
| **GROUP** | **Mean body weight** | **Mean final body weight** | **Body weight increment** |
| **GnRH-TT** | 10.46 ± 0.41 | 55.00 ± 0.86 | 44.54 |
| **GnRHm1-TT** | 12.80 ± 0.35 | 59.50 ± 1.80 | 46.70 |
| **GnRH-BSA** | 13.33 ± 0.31 | 57.67 ± 8.50 | 44.34 |
| **GnRHm1-BSA** | 12.67 ± 0.59 | 54.00 ± 5.29 | 41.33 |
| **Placebo** | 15.83 ± 1.26 | 63.00 ± 6.56 | 47.17 |
| **FECHA** | 9 Enero 1996 | 29 Abril 1996 | |
| **Age in weeks** | 11-12 | 27-28 | |

d)

**Table 4**

| Results of the test of primary reflex of sexual intercourse of immunized piglets. | | | | | | | | | | | | | |
|---|---|---|---|---|---|---|---|---|---|---|---|---|---|
| **GROUP** | **ANIMAL** | **Approaching** | | | **Covering** | | | **Erection** | | | **Ejaculation** | | |
| | | **1** | **2** | **3** | **1** | **2** | **3** | **1** | **2** | **3** | **1** | **2** | **3** |
| GnRH-TT. | 20 | G | G | G | G | G | N | NO | NO | NO | NO | NO | NO |
| | 30 | G(*) | G(*) | M | N | N | N | NO | NO | NO | NO | NO | NO |
| | 36 | G | N | N | G | N | N | NO | NO | NO | NO | NO | NO |
| GnRHm1-TT. | 15 | M | M | N | N | N | N | NO | NO | NO | NO | NO | NO |
| | 24 | M | M | M | N | N | N | NO | NO | NO | NO | NO | NO |
| | 37 | M | G(*) | G | N | N | N | NO | NO | NO | NO | NO | NO |
| GnRH-BSA. | 2 | G | G | G | G | G | G | NO | NO | NO | NO | NO | NO |
| | 23 | G | G | G | G | G | G | NO | NO | NO | NO | NO | NO |
| | 25 | G | G | G | G | G | G | NO | YES | YES | NO | YES | NO |
| GnRHm1-BSA. | 14 | G | G | M | N | N | N | YES | NO | NO | NO | NO | NO |
| | 16 | G | G | G | G | G | G | NO | NO | NO | NO | NO | NO |
| | 39 | M | G | G | M | G | G | NO | NO | NO | NO | NO | NO |
| Placebo. | 1 | G | G(*) | G | G | G | G | NO | NO | YES | NO | NO | YES |
| | 11 | G | G | G | G | N | N | NO | NO | NO | NO | NO | NO |
| | 17 | G | G | G | G | G | G | NO | YES | YES | NO | NO | YES |
| a) approaching: G - good, G(*)- aggressive approaching, M - medium, N - means not approaching, b) covering: G - good reflex, M- medium reflex, N - not reflexs. | | | | | | | | | | | | | |

### EXAMPLE 4

### Evaluation after the slaughter

After the slaughter, the testicles were excised and the epididymes removed. The testicles were measured and weighed and the epididymes were weighed. Two samples of testicular and epididymal tissue were fixed for histology. After fixing the median part of every transversal section of these tissues, the samples were embedded in paraffin and cut in 5 µm sections. The staining was performed with hematoxylin and eosin. a)

**Table 5**

| Measurement of testicles and weights of epididymes and testicles | | | | | | | |
|---|---|---|---|---|---|---|---|
| **GROUP** | **ANIMAL** | **Size of testicles, mm (length X wide)** | | **Epididymes weight, In grams** | | **Testicles weigth, in grams.** | |
| | | **Right** | **Left** | **Right** | **Left** | **Right** | **Left** |
| **GnRH-TT** | 20 | 67x42 | 69x41 | 31.69 | 31.56 | 57.60 | 63.37 |
| | 30 | 48x27 | 50x28 | 17.42 | 17.78 | 19.46 | 20.86 |
| | 36 | 57x37 | 64x38 | 26.21 | 28.11 | 36.46 | 43.60 |
| **GnRHm1-TT** | 15 | 64x31 | 57x29 | 19.34 | 15.84 | 32.53 | 26.30 |
| | 24 | 48x30 | 51x38 | 27.10 | 27.67 | 28.55 | 32.63 |
| | 37 | 44x26 | 43x27 | 14.55 | 13.80 | 13.96 | 14.22 |
| **GnRH-BSA** | 2 | 65x40 | 64x40 | 28.50 | 27.74 | 51.47 | 50.21 |
| | 23 | 106x59 | 101x58 | 48.38 | 45.23 | 178.30 | 180.59 |
| | 25 | 67x44 | 67x44 | 36.87 | 36.25 | 66.44 | 66.50 |
| **GnRHm1-BSA** | 14 | 89x51 | 87x52 | 44.31 | 43.84 | 125.00 | 122.66 |
| | 16 | 82x51 | 81x48 | 30.42 | 29.51 | 100.07 | 93.03 |
| | 39 | 74x43 | 77x43 | 34.54 | 30.40 | 67.33 | 72.36 |
| **Placebo** | 1 | 82x52 | 90x55 | 53.11 | 58.42 | 116.33 | 125.23 |
| | 11 | 80x54 | 81x52 | 34.35 | 35.04 | 114.00 | 111.23 |
| | 17 | 92x68 | 94x59 | 53.28 | 51.23 | 159.24 | 163.61 |

From the results in previous table were calculated the rates testicles weight /body weight:

**Table 6**

| **Testicles Weight/body weight rates** | | | | | |
|---|---|---|---|---|---|
| ***** | **Placebo** | **GnRH-BSA** | **GnRHm1-BSA** | **GnRH-TT** | **GnRHm1-TT** |
| **Rigth testicles** | 2.041 | 0.804 | 2.232 | 1.067 | 0.561 |
| | 1.839 | 2.923 | 2.085 | 0.351 | 0.484 |
| | 2.275 | 1.384 | 1.161 | 0.657 | 0.227 |
| **Left testicles** | 2.197 | 0.785 | 2.190 | 1.174 | 0.453 |
| | 1.794 | 2.960 | 1.938 | 0.376 | 0.553 |
| | 2.337 | 1.385 | 1.248 | 0.786 | 0.231 |
| **Media** | 2.081 | 1.707 | 1.809 | 0.735 | 0.418 |

| | | | | | |
|---|---|---|---|---|---|
| ^{*}All values were multipled by 10³ | | | | | |

For every column on previous table were calculated the probabilities associated to the Student test:

**Table 7**

| **Probabilities associated to the Student test of values in table 6** | | | | |
|---|---|---|---|---|
| | **GnRH-BSA** | **GnRHm1-BSA** | **GnRH-TT** | **GnRHm1-TT** |
| **Placebo** | 0.194992 | 0.119628 | 5.84x10⁻⁶ | 1.9x10⁻⁸ |
| **GnRH-BSA** | | 0.412618 | 0.023363 | 0.005215 |
| **GnRHm1-BSA** | | | 0.000608 | 2.48x10⁻⁵ |
| **GnRH-TT** | | | | 0.032757 |

Note: there is statistical significant difference when associated probability is < 0.05.
There is no statistical significant difference between the placebo group and the two groups conjugated to BSA. For the contrary, between whatever of these three groups and the groups conjugated to the tetanus toxoide there is statistical significant differences. And between these two group there is also significant difference, being the media of the GnRHm1-TT the smallest among al the groups and therefore the one with a major reduction of testicle size.
The same procedure was aplied to epididyme data.

**Table 8**

| **Epididyme Weight/body weight rates** | | | | | |
|---|---|---|---|---|---|
| | **Placebo** | **GnRH-BSA** | **GnRHm1-BSA** | **GnRH-TT** | **GnRHm1-TT** |
| **Rigth epididyme** | 0.932 | 0.445 | 0.791 | 0.587 | 0.333 |
| | 0.554 | 0.793 | 0.634 | 0.314 | 0.459 |
| | 0.761 | 0.768 | 0.596 | 0.472 | 0.237 |
| **Left epididyme** | 1.025 | 0.433 | 0.782 | 0.584 | 0.273 |
| | 0.565 | 0.741 | 0.614 | 0.320 | 0.469 |
| | 0.732 | 0.755 | 0.524 | 0.506 | 0.224 |
| **Media** | 0.761 | 0.656 | 0.657 | 0.464 | 0.333 |

The same way was calculated the probability matrix.

**Table 9**

| **Probabilities associated to the Student test of values in table 8** | | | | |
|---|---|---|---|---|
| | **GnRH-BSA** | **GnRHm1-BSA** | **GnRH-TT** | **GnRHm1-TT** |
| **Placebo** | 0.16702 | 0.134319 | 0.004564 | 0.000365 |
| **GnRH-BSA** | | 0.495529 | 0.02377 | 0.001373 |
| **GnRHm1-BSA** | | | 0.007825 | 0.000201 |
| **GnRH-TT** | | | | 0.038709 |

With epididymes were obtained the same results as with testicles.

The results obtained with the peptides GnRH y GnRHm1 conjugated to BSA are very similar to those of the placebo group. This behaviour can be explained by the high degree of homology (80%) between the bovine and porcine albumins, being BSA seen as a self protein by pig's immune system.
When GnRHm1-TT and pacebo group are compared a reduction of 2,3 times of the weigth of the epididymes and 5,0 times of the testicle weigth is observed. Comparing GnRH-TT and placebo groups a reduction of 1,6 times in the weigth of epididymes and 2,4 times the weigth of the testicles.
As conclusion a new GnRH-analog peptide has been designed, which shows to be 1,4 to 1,8 time more efective than natural GnRH when both are conjugated to the universal epitope of the tetanus toxoide.

### DESCRIPTION OF FIGURES

**Figure 1:** Testicles from placebo (left), GnRH-TT (center) y GnRHm1-TT (rigth) groups. It is evident the effect produced in the groups conjugated to TT. Between the last two groups is observed a a higher reduction of tescicle size in the GnRHm1-TT group.
**Figure 2.** Cromatograms off peptides a)GnRH, b)GnRHm1, c)GnRH-TT y d) GnRHm1-TT. In all cases was used a reverse fase column C18 VYDAC (4,6 x 100 mm) with a gradient of 5-60% of acetonitrile(0,05 % of TFA) in water (0,1 % of TFA). The peptides were detected at 226 nm wave length.

## Claims

1. A peptide that inhibits the release of gonadotropins, having the sequence:
Glu-His-Trp-Ser-Tyr-Pro-Leu-Arg-Pro-Gly

2. A peptide acording to claim 1 composed only of L-aminoacids.

3. A peptide or protein having as part of its aminoacid sequence the sequence described in claim 1.

4. A vaccine comprising a peptide or protein acording to claims 1,2 and 3 suitable for human or veterinary use.


SEQUENCE LISTING

(1) GENERAL INFORMATION:
(i) APPLICANT:
(A) NAME: CENTRO DE INGENIERIA GENETICA Y
BIOTECNOLOGIA
(B) STREET: Ave. 31 entre 158 y 190, Playa
(C) CITY: Ciudad de La Habana
(E) COUNTRY: Cuba
(F) POSTAL CODE (ZIP): 10600
(G) TELEPHONE: 53 7 218466
(H) TELEFAX: 53 7 218070/336008

(ii) TITLE OF INVENTION: VACCINE PREPARATION FOR THE
REVERSIBLE IMMUNOCASTRATION OF MAMMALS.

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30
(EPO)
(vi) PRIOR APPLICATION DATA:
(A) APPLICATION NUMBER: CU 120/96

(B) FILING DATE: 17-DEC-1996

(2) INFORMATION FOR SEQ ID NO: 1:
(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide
(iii) HYPOTHETICAL: NO
(iv) ANTI-SENSE: NO

(vi) ORIGINAL SOURCE:
(A) ORGANISM: Gonadotropin releasing hormone mutant
(C) INDIVIDUAL ISOLATE: Mammals

(xi) SEQUENCE DESCRIPTION: SEQ ID NO. 1:

Glu His Trp Ser Tyr Pro Leu Arg Pro Gly
1               5                   10